# EUROPEAN PATENT APPLICATION

(11) **EP 4 674 424 A1**
(43) Date of publication of application: **07.01.2026**
(21) Application number: 24764093.1
(22) Date of filing: 30.01.2024
(51) Int. Cl.: A61K 38/08, A61P 43/00, A61P 3/00, A61P 35/00, A61P 25/28, A23L 33/18, A61K 8/64, A61Q 19/08

(54) **ANTI-AGING COMPOSITION COMPRISING PEPTIDE COMPLEX AS ACTIVE INGREDIENT**

(30) Priority: 28.02.2023 KR 20230027382
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); KIM, Seon Soo, Anyang-si, Gyeonggi-do 14119 (KR)
(74) Representative: Regimbeau
(86) International application number: PCT/KR2024/001429
(87) International publication number: WO 2024/181693

(57) **Abstract**

A peptide complex of the present invention promotes the expression of an SIRT1 gene, which is known as a longevity gene, promotes the expression of an FGF21 gene and increases the phosphorylation of AMPK. In addition, a peptide complex of the present invention exhibits an anti-inflammatory effect. Therefore, the peptide complex of the present invention inhibits aging, promotes the expression of SIRT1 gene, which is a longevity gene, and can be used for treating or preventing SIRT1-related diseases.

## Description

### TECHNICAL FIELD

### [Cross-reference to Related Applications]

This application claims the benefit of Korean Patent Application No. 10-2023-0027382, filed on February 28, 2023, in the Korean Intellectual Property Office, the disclosure of which is incorporated herein in its entirety by reference.

### [Technical Field]

The present invention relates to an anti-aging composition containing a peptide complex as an active ingredient.

### BACKGROUND ART

Sirtuin 1 (SIRT1, silent mating type information regulation 2 homolog), also known as a longevity gene, is also known as NAD-dependent deacetylase sirtuin-1 and has been recognized as an important factor related to aging in cells through calorie restriction. In yeast, it is known to play an important role in the process of extending the lifespan of yeast due to calorie restriction as silent information regulator 2 (SIR2), and its homologous gene in mammals is the SIRT1 gene. In mammals, SIRT1 is also well known to play a role of deacetylation and a role of inactivating the p53 protein. Aging may also be classified as a chronic, very low-level inflammatory response, and NF-κB, which plays an important role in this process, is a transcription factor regulated by SIRT1 and is a major element of the inflammatory response. Since it is expected that substances that activate the SIRT1 gene will be able to inhibit cellular aging and become treatments for serious diseases, studies are currently underway to develop SIRT1 activating agents derived from various sources.

Meanwhile, WO 2018/183882 (PCT/US2018/025450) discloses a peptide having an activity of regulating the expression of sirtuin genes in skin cells.

### [Prior Art Documents]

### [Patent Documents]

WO 2018/183882 (PCT/US2018/025450)

### DISCLOSURE OF THE INVENTION

### TECHNICAL PROBLEM

As a result of the research efforts to develop safer and more effective anti-aging active ingredients, the present inventors have experimentally confirmed that a complex of two peptides of the present invention effectively activates the SIRT1 gene, an intracellular aging mediator, thereby completing the present invention.

Accordingly, an object of the present invention is to provide an anti-aging composition containing a peptide complex as an active ingredient.

Another object of the present invention is to provide a composition for activating sirtuin 1 (SIRT1) containing a peptide complex as an active ingredient.

Still another object of the present invention is to provide a pharmaceutical composition for treating or preventing sirtuin 1 (SIRT1)-related diseases, containing a peptide complex as an active ingredient.

### TECHNICAL SOLUTION

An aspect of the present invention provides an anti-aging composition which contains, as an active ingredient, a peptide complex containing (i) a peptide containing an amino acid sequence of SEQ ID NO: 1 and (ii) a peptide containing an amino acid sequence of SEQ ID NO: 2.

Another aspect of the present invention provides a composition for activating sirtuin 1 (SIRT1), which contains, as an active ingredient, a peptide complex containing (i) a peptide containing an amino acid sequence of SEQ ID NO: 1 and (ii) a peptide containing an amino acid sequence of SEQ ID NO: 2.

Still another aspect of the present invention provides a pharmaceutical composition for treating or preventing a sirtuin 1 (SIRT1)-related disease, which contains, as an active ingredient, a peptide complex containing (i) a peptide containing an amino acid sequence of SEQ ID NO: 1 and (ii) a peptide containing an amino acid sequence of SEQ ID NO: 2.

The present invention is described in detail below.

### 1. Peptide complex and activity thereof

The present invention discloses a peptide complex, which contains, as an active ingredient for anti-aging use, (i) a peptide containing an amino acid sequence disclosed in SEQ ID NO: 1 and (ii) a peptide containing an amino acid sequence disclosed in SEQ ID NO: 2.

The term "peptide" as used herein means a linear molecule formed by amino acid residues that are joined each other by peptide bonds.

The peptide containing the amino acid sequence of SEQ ID NO: 1 of the present invention or the peptide containing the amino acid sequence of SEQ ID NO: 2 of the present invention may be used without modification, but a variant or fragment of an amino acid having a different sequence by deletion, insertion, substitution, or a combination thereof of amino acid residues may be used within a range that does not affect the original activities of the peptide, such as an anti-aging activity and an SIRT1 activation activity.

The peptide of the present invention may be modified by phosphorylation, sulfation, acrylation, glycosylation, methylation, farnesylation, etc., within a range that does not change its activity.

The peptide of the present invention includes a peptide containing an amino acid sequence substantially identical to a peptide containing an amino acid sequence of SEQ ID NO: 1 or a peptide containing an amino acid sequence of SEQ ID NO: 2, and a variant or an active fragment thereof. The substantially identical amino acid sequence means an amino acid sequence having a sequence identity of 75% or more, for example, 80% or more, 85% or more, 90% or more, 95% or more, or 97% or more, with the amino acid sequence of SEQ ID NO: 1 or the amino acid sequence of SEQ ID NO: 2, respectively. In addition, the peptide may further include a targeting sequence, a tag, a labeled residue, an amino acid sequence prepared for a specific purpose of increasing half-life or stability of a peptide.

The peptide of the present invention may be one in which a portion of the amino acid sequence is selected and N-terminal and/or C-terminal modifications are induced to increase its activity. Through such N-terminal and/or C-terminal modifications, the stability of the peptide of the present invention may be significantly improved, and for example, the half-life of the peptide when administered *in vivo* may be increased. The term "stability" includes not only stability *in vivo* that protects the peptide of the present invention from attack by proteases *in vivo,* but also storage stability *(e.g.,* room temperature storage stability).

The N-terminal modification may be a modification in which a protecting group selected from the group consisting of an acetyl group, a fluoreonylmethoxycarbonyl group, a formyl group, a palmitoyl group, a myristyl group, a stearyl group, and polyethylene glycol (PEG) is bonded to the N-terminus of the peptide. The C-terminal modification may be a modification in which a hydroxyl group (-OH), an amino group (-NH₂), an azide group (-NHNH₂), etc. is bonded to the C-terminus of the peptide, but is not limited thereto.

The peptide of the present invention may be prepared by various methods widely known in the art to which the present invention pertains. For example, the peptide of the present invention may be prepared by chemical synthesis methods known in the art, particularly solid-phase synthesis techniques (Merrifield, J. Amer. Chem. Soc. 85:2149-54(1963); Stewart, et al., Solid Phase Peptide Synthesis, 2nd. ed., Pierce Chem. Co.: Rockford, 111(1984)) or liquid-phase synthesis techniques (U.S. Patent No. 5,516,891).

The peptide complex of the present invention contains a peptide containing the amino acid sequence of SEQ ID NO: 1 and a peptide containing the amino acid sequence of SEQ ID NO: 2.

The peptide complex of the present invention may mean a mixture, in which a peptide containing the amino acid sequence of SEQ ID NO: 1 and a peptide containing the amino acid sequence of SEQ ID NO: 2 described above are mixed.

In the peptide complex of the present invention, the ratio of the peptide containing the amino acid sequence of SEQ ID NO: 1 and the peptide containing the amino acid sequence of SEQ ID NO: 2 is not limited to a specific range, and for example, the ratio may be used by selecting an appropriate range within the range of a weight ratio of 1 : 0.1-100.

The peptide complex of the present invention has anti-aging activity.

The peptide complex of the present invention has an activity of inhibiting cellular aging.

The peptide complex of the present invention has an activity of increasing the expression level of sirtuin 1 (SIRT1) protein or fibroblast growth factor 21 (FGF21) protein in a cell, or increasing the phosphorylation of AMP-activated protein kinase (AMPK).

In a specific embodiment, the cell may be an adipocyte or myocyte.

The peptide complex of the present invention may increase the expression of sirtuin 1 (SIRT1) gene, fibroblast growth factor 21 (FGF21) gene, AMP-activated protein kinase (AMPK) gene, or FOXO3A gene in a cell.

In a specific embodiment, the cell may be an adipocyte or myocyte.

The peptide complex of the present invention has anti-inflammatory activity.

The peptide complex of the present invention induces a decrease in phosphorylation of IκBα or a decrease in the expression level of the inflammatory cytokine TNFα in inflammation-induced cells.

In a specific embodiment, the inflammation-induced cell may be an adipocyte or myocyte.

The peptide complex of the present invention has an activity of activating the sirtuin 1 (SIRT1) gene.

The peptide complex of the present invention has an activity of activating the AMPK gene.

The AMPK activated by the peptide complex of the present invention may generate a feedback loop by phosphorylation/activation through SIRT1-mediated deacetylation, and AMPK may inhibit glycogen synthesis, oxidation of fatty acids, HMG-Co-A reductase, and mTOR functions through its downstream signaling. Reduced mTORC1 inhibits apoptosis through increased autophagy, and activated SIRT1 deacetylates the RelA/p65 component of NFkB to prevent the degradation of IkB and sequester NF-kB in the cytoplasm to thereby inhibit the expression of pro-inflammatory genes. The peptide complex of the present invention increases the expression of SIRT1 and FOXO3 proteins and promotes the phosphorylation of AMPK, and as a result, the peptide complex of the present invention is expected to have the anti-aging and longevity effects.

### 2. Composition for anti-aging

According to an aspect of the present invention, there is provided an anti-aging composition, which contains a peptide complex, as an active ingredient, containing (i) a peptide containing an amino acid sequence of SEQ ID NO: 1 and (ii) a peptide containing an amino acid sequence of SEQ ID NO: 2.

The peptide complex of the present invention has anti-aging activity the action of increasing the expression level of sirtuin 1 (SIRT1) protein or fibroblast growth factor 21 (FGF21) protein in cells, or the action of increasing the phosphorylation of AMP-activated protein kinase (AMPK).

In addition, the peptide complex of the present invention has anti-aging activity by inducing a decrease in phosphorylation of IκBα or a decrease in the expression level of the inflammatory cytokine TNFα in cells under an inflammatory response state.

In an embodiment, the anti-aging composition may be a composition for use in inhibition of cellular aging.

In an embodiment, the cell may be an adipocyte or myocyte.

In an embodiment, the cell may be an adipocyte or myocyte in a state of insulin metabolic disorder or in an inflammatory state.

In an embodiment, the insulin metabolic disorder may refer to a state of having insulin resistance.

In an embodiment, the anti-aging composition of the present invention may be a pharmaceutical composition for use in inhibiting of aging.

In an embodiment, the aging may be cellular aging.

In an embodiment, the cell can be an adipocyte or myocyte.

In an embodiment, the cell may be an adipocyte or myocyte in a state of insulin metabolic disorder or in an inflammatory state.

In an embodiment, the insulin metabolic disorder may refer to a state of having insulin resistance.

In an embodiment, the aging may be cellular aging induced by oxidative stress. The pharmaceutical composition of the present invention may contain a therapeutically effective amount of the peptide complex and a pharmaceutically acceptable carrier.

The term "therapeutically effective amount" means an amount sufficient for the peptide complex, which is an active ingredient of the pharmaceutical composition of the present invention, to achieve its activity or efficacy, for example, an amount sufficient to achieve the efficacy of inhibiting aging.

The pharmaceutically acceptable carriers mentioned above are those commonly used in the preparation of formulations and include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, mineral oil, *etc.*

The pharmaceutical composition of the present invention may additionally include, in addition to the above-described components, a lubricant, a wetting agent, a sweetener, a flavoring agent, an emulsifier, a suspending agent, a preservative, etc., but is not limited thereto.

Suitable pharmaceutically acceptable carriers and formulations are described in detail in Remington: The Science and Practice of Pharmacy, (19th ed., 1995, Williams & Wilkins).

The pharmaceutical composition of the present invention may be administered by any suitable route for inhibiting aging, for example, it may be administered orally or parenterally, and in the case of parenteral administration, it may be administered by intravenous injection, subcutaneous injection, intramuscular injection, intraperitoneal injection, topical administration, transdermal administration, *etc.*

The administration dose of the pharmaceutical composition may be, but is not limited to, 0.0001 µg to 100 mg, 0.001 µg to 100 mg, 0.01 µg to 100 mg, 0.1 µg to 100 mg, or 1.0 µg to 1000 mg per day, and may be prescribed in various ways depending on factors such as the formulation method, administration method, patient's age, weight, sex, pathological condition, food, administration time, administration route, excretion rate, and reaction sensitivity.

The pharmaceutical composition of the present invention may be prepared in a unit dose form or may be prepared by placing it in a multi-dose container by formulating it using a pharmaceutically acceptable carrier and/or excipient according to a method that may be easily performed by a person having ordinary skill in the art to which the present invention pertains. In particular, the formulation may be in the form of a solution, suspension, or emulsion in an oil or aqueous medium, or in the form of an extract, powder, granules, tablets, or capsules, and may additionally contain a dispersant or stabilizer.

In an embodiment, the anti-aging composition of the present invention may be a food composition for use in the inhibition of aging. That is, the present invention provides a food composition for inhibiting aging, which contains a peptide complex, as an active ingredient, containing (i) a peptide containing the amino acid sequence of SEQ ID NO: 1; and (ii) a peptide containing the amino acid sequence of SEQ ID NO: 2.

In an embodiment, the anti-aging food composition may be a functional food composition for anti-aging.

In an embodiment, the aging may be cellular aging.

In an embodiment, the cell can be an adipocyte or myocyte.

In an embodiment, the cell may be an adipocyte or myocyte in a state of insulin metabolic disorder or in an inflammatory state.

In an embodiment, the insulin metabolic disorder may be a state of having insulin resistance.

In an embodiment, the aging may be cellular aging induced by oxidative stress.

In the functional food composition of the present invention, the peptide complex may be contained in an appropriate amount selected within the range of 0.0001 wt% to 10 wt% with respect to the total composition weight.

In an embodiment, the functional food composition of the present invention may contain a sitologically effective amount of the peptide complex and a sitologically acceptable carrier.

The food composition of the present invention contains not only the peptide complex as the active ingredient, but also components commonly added during food manufacturing, and may contain, for example, proteins, carbohydrates, fats, nutrients, seasonings, and flavoring agents. Examples of the carbohydrates described above may include monosaccharides, for example, glucose, fructose, *etc.;* disaccharides, for example, maltose, sucrose, oligosaccharides, *etc.;* and polysaccharides, for example, dextrin, cyclodextrin, *etc.,* and sugar alcohols, for example, xylitol, sorbitol, erythritol, *etc.* As flavoring agents, natural flavoring agents, thaumatin, stevia extracts (for example, rebaudioside A, glycyrrhizin, *etc.),* and synthetic flavoring agents (saccharin, aspartame, *etc*.) may be used. The proportion of the carbohydrate may generally be about 1 g to 20 g, preferably about 5 g to 12 g, per 100 g of the food composition of the present invention, but is not limited thereto.

The functional food composition of the present invention may contain, in addition to the above-described ingredients, various nutrients, vitamins, minerals (electrolytes), flavoring agents such as synthetic flavoring agents and natural flavoring agents, coloring agents and thickeners (cheese, chocolate, *etc.),* pectic acid and its salts, alginic acid and its salts, organic acids, protective colloid thickeners, pH regulators, stabilizers, preservatives, glycerin, alcohol, carbonating agents used in carbonated beverages, *etc.* In addition, it may contain fruit pulp for the production of natural fruit juice and fruit juice drinks and vegetable drinks.

For example, when the functional food composition of the present invention is prepared as a drink, in addition to the peptide complex, which is an active ingredient of the present invention, citric acid, liquid fructose, sugar, glucose, acetic acid, malic acid, juice, a *Eucommia* extract, a jujube extract, a licorice extract, *etc.* may be additionally contained.

In an embodiment, the anti-aging composition of the present invention may be a cosmetic composition for use in the inhibition of aging. That is, the present invention provides an anti-aging cosmetic composition which contains a peptide complex, as an active ingredient, containing: (i) a peptide containing the amino acid sequence of SEQ ID NO: 1; and (ii) a peptide containing the amino acid sequence of SEQ ID NO: 2.

In an embodiment, the aging may be cellular aging induced by oxidative stress.

In an embodiment, the aging may be aging of skin cells.

The cosmetic composition may be prepared into any formulation commonly prepared in the technical field to which the present invention belongs, and may be an agent for external skin. For example, it may be formulated into a solution, suspension, emulsion, paste, gel, cream, lotion, powder, soap, surfactant-containing cleansing, oil, powder foundation, emulsion foundation, wax foundation, spray, *etc.,* but is not limited thereto.

The cosmetic composition may be prepared in various forms, such as a solution, sol-gel, emulsion, oil, wax, and aerosol, such as a softening toner, a nourishing toner, a nourishing cream, a massage cream, an essence, an eye cream, a cleansing cream, a cleansing foam, a cleansing water, a pack, a spray, a powder, a hair tonic, a hair cream, a hair lotion, a hair shampoo, a hair rinse, a hair conditioner, a hair spray, a hair aerosol, a pomade, and a gel, but is not limited thereto.

The cosmetic composition of the present invention may contain other additives such as excipients and carriers, and it is possible to apply and mix as much as necessary the common ingredients mixed in general skin cosmetics.

When the formulation of the cosmetic composition is in the form of a paste, cream, or gel, animal oil, vegetable oil, wax, paraffin, starch, tragacanth, cellulose derivatives, polyethylene glycol, silicone, bentonite, silica, talc, zinc oxide, etc. may be used as a carrier component.

When the formulation of the above cosmetic composition is in the form of a powder or spray, lactose, talc, silica, aluminum hydroxide, calcium silicate, or polyamide powder may be used as a carrier component, and particularly in the case of a spray, a propellant such as chlorofluorohydrocarbon, propane/butane or dimethyl ether may be additionally contained, but is not limited thereto.

When the formulation of the above cosmetic composition is a solution or emulsion, as a carrier component, a solvent, a solubilizer, or an emulsifier may be used, and for example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol, a fatty acid ester of sorbitan, etc. may be used.

When the formulation of the cosmetic composition is in the form of a suspension, as a carrier component, liquid diluents such as water, ethanol, or propylene glycol; suspending agents such as ethoxylated isostearyl alcohol, polyoxyethyl sorbitol ester, and polyoxyethylene sorbitan ester; microcrystalline cellulose, aluminum metahydroxide, bentonite, agar, tragacanth, etc. may be used.

When the formulation of the cosmetic composition is a surfactant-containing cleansing, as a carrier component, fatty alcohol sulfate, fatty alcohol ether sulfate, sulfosuccinic acid monoester, isethionate, an imidazolinium derivative, methyl taurate, sarcosinate, fatty acid amide ether sulfate, alkyl amidobetaine, fatty alcohol, fatty acid glyceride, fatty acid diethanolamide, vegetable oil, a lanolin derivative, an ethoxylated glycerol fatty acid ester, *etc.* may be used.

When the formulation of the cosmetic composition is a hair shampoo, base components for forming a shampoo, such as a thickener, a surfactant, a viscosity regulator, a moisturizer, a pH regulator, a preservative, an essential oil, *etc.* may be mixed with the peptide of the present invention. CDE may be used as the thickener; LES, which is an anionic surfactant, and coco betaine, which is an amphoteric surfactant, may be used as the surfactant; polyquat may be used as the viscosity regulator; glycerin may be used as the moisturizer; and citric acid or sodium hydroxide may be used as the pH regulator. A grapefruit extract, etc. may be used as the preservative, and in addition thereto, essential oils such as cedarwood, peppermint, and rosemary, silk amino acids, pentaol, or vitamin E may be added.

The ingredients to be contained in the cosmetic composition may further include, as active ingredients, in addition to the peptide of the present invention and the carrier component, ingredients commonly used in cosmetic compositions, for example, conventional auxiliary agents such as antioxidant, a stabilizer, a solubilizer, vitamins, pigments, and fragrances, but are not limited thereto.

The peptide of the present invention may be contained in the composition described above at a concentration of 0.01 µM to 1000 µM, and specifically, the peptide of the present invention may be contained in the composition described above at a concentration of 0.01 µM to 1000 µM; 0.05 µM to 800 µM, 0.05 µM to 700 µM, 0.05 µM to 600 µM, 0.05 µM to 500 µM, 0.05 µM to 300 µM, 0.05 µM to 200 µM; 0.1 µM to 800 µM, 0.1 µM to 700 µM, 0.1 µM to 600 µM, 0.1 µM to 500 µM, 0.1 µM to 300 µM, 0.1 µM to 200 µM; 1 µM to 800 µM, 1 µM to 700 µM, 1 µM to 600 µM, 1 µM to 500 µM, 1 µM to 300 µM, 1 µM to 200 µM; 5 µM to 800 µM, 5 µM to 700 µM, 5 µM to 600 µM, 5 µM to 500 µM, 5 µM to 300 µM, or 5 µM to 200 µM, but is not limited thereto.

### 3. Composition for activating sirtuin 1 (SIRT1) and composition for treating or preventing SIRT1-related diseases

According to another aspect of the present invention, the present invention provides a composition for activating sirtuin 1 (SIRT1), which contains a peptide complex, as an active ingredient, containing (i) a peptide containing the amino acid sequence of SEQ ID NO: 1 and (ii) a peptide containing the amino acid sequence of SEQ ID NO: 2.

In an embodiment, the activation of sirtuin 1 (SIRT1) means promoting or enhancing the expression of the sirtuin 1 (SIRT1) gene.

According to still another aspect of the present invention, there is provided a method for activating a sirtuin 1 (SIRT1) gene, which includes administering to a subject in need of sirtuin 1 (SIRT1) activation a peptide complex containing (i) a peptide containing the amino acid sequence of SEQ ID NO: 1 and (ii) a peptide containing the amino acid sequence of SEQ ID NO: 2.

In an embodiment, the activation of sirtuin 1 (SIRT1) means promoting or enhancing the expression of the sirtuin 1 (SIRT1) gene.

In an embodiment, the activation of sirtuin 1 (SIRT1) may be activation of SIRT1 in a cell.

In an embodiment, the cell may be an adipocyte or myocyte.

In an embodiment, the cell may be an adipocyte or myocyte in a state of insulin metabolic disorder or an inflammatory state.

In an embodiment, the insulin metabolic disorder may be a state of having insulin resistance.

According to still another aspect of the present invention, there is provided a pharmaceutical composition for treating or preventing a sirtuin 1 (SIRT1)-related disease, which contains a peptide complex containing (i) a peptide containing the amino acid sequence of SEQ ID NO: 1 and (ii) a peptide containing the amino acid sequence of SEQ ID NO: 2 as an active ingredient.

In an embodiment, the sirtuin 1 (SIRT1)-related disease refers to a disease that can be treated by promoting or enhancing the expression of sirtuin 1 (SIRT1) gene.

In an embodiment, the sirtuin 1 (SIRT1)-related disease may be a disease selected from the group consisting of, for example, metabolic diseases, cancer, and neurodegenerative diseases.

In an embodiment, the metabolic disease may be a disease selected from the group consisting of insulin-resistant diabetes, adult-onset diabetes, diabetic nephropathy, type 2 diabetes, obesity, impaired glucose tolerance, hypercholesterolemia, dyslipidemia, hyperlipidemia, microvascular dysfunction, and arteriosclerosis.

In an embodiment, the cancer may be a cancer selected from the group consisting of blood cancer, breast cancer, head or neck cancer, uterine cancer, cervical cancer, ovarian cancer, lung cancer, bladder cancer, esophageal cancer, mesothelioma, neuroblastoma, testicular cancer, leukemia, stomach cancer, liver cancer, colon cancer, skin cancer, brain cancer, laryngeal cancer, prostate cancer, thyroid cancer, kidney cancer, and rectal cancer.

In an embodiment, the neurodegenerative disease may be a disease selected from the group consisting of Alzheimer's disease, Huntington's disease, Parkinson's disease, amyotrophic lateral sclerosis, multiple sclerosis, diseases caused by polyglutamine aggregation, AIDS-related dementia, sensory neuropathy, autonomic neuropathy, motor neuropathy, and retinopathy.

The pharmaceutical composition may contain a therapeutically effective amount of the peptide complex and a pharmaceutically acceptable carrier.

The term "therapeutically effective amount" means an amount sufficient for the peptide complex, which is an active ingredient of the pharmaceutical composition of the present invention, to achieve its activity or efficacy, for example, an amount sufficient to achieve efficacy in treating or preventing a sirtuin 1 (SIRT1)-related disease.

The pharmaceutically acceptable carriers mentioned above are those commonly used in the preparation of formulations and include, but are not limited to, lactose, dextrose, sucrose, sorbitol, mannitol, starch, acacia gum, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate, and mineral oil.

The pharmaceutical composition of the present invention may further include, in addition to the components, a lubricant, a wetting agent, a sweetener, a flavoring agent, an emulsifier, a suspending agent, a preservative, *etc.,* but is not limited thereto.

Suitable pharmaceutically acceptable carriers and formulations are described in detail in Remington: The Science and Practice of Pharmacy, (19th ed., 1995, Williams & Wilkins).

The pharmaceutical composition of the present invention may be administered by any suitable route for treating a sirtuin 1 (SIRT1)-related disease, for example, may be administered orally or parenterally, and in the case of parenteral administration, it may be administered by intravenous injection, subcutaneous injection, intramuscular injection, intraperitoneal injection, topical administration, transdermal administration, etc.

The administration dose of the pharmaceutical composition may be, but is not limited to, 0.0001 µg to 100 mg, 0.001 µg to 100 mg, 0.01 µg to 100 mg, 0.1 µg to 100 mg, or 1.0 µg to 1000 mg per day, and may be prescribed in various ways depending on factors such as the formulation method, administration method, patient's age, weight, sex, pathological condition, food, administration time, administration route, excretion rate, and reaction sensitivity.

The pharmaceutical composition of the present invention may be prepared in a unit dose form or may be prepared by placing it in a multi-dose container by formulating it using a pharmaceutically acceptable carrier and/or excipient according to a method that can be easily performed by a person having ordinary skill in the art to which the present invention pertains. In particular, the formulation may be in the form of a solution, suspension, or emulsion in an oil or aqueous medium, or in the form of an extract, powder, granules, tablets, or capsules, and may additionally contain a dispersant or stabilizer.

### 4. Use of peptide complex of present invention

According to an aspect of the present invention, there is provided a method for inhibiting aging, which includes administering to a subject, in need of inhibition of aging, an effective amount of a peptide complex containing (i) a peptide containing the amino acid sequence of SEQ ID NO: 1 and (ii) a peptide containing the amino acid sequence of SEQ ID NO: 2.

According to another aspect of the present invention, there is provided a use, in the inhibition of aging, of a peptide complex, which contains (i) a peptide containing the amino acid sequence of SEQ ID NO: 1 and (ii) a peptide containing the amino acid sequence of SEQ ID NO: 2.

According to another aspect of the present invention, there is provided a use, in the inhibition of aging, of a composition which contains a peptide complex containing (i) a peptide containing the amino acid sequence of SEQ ID NO: 1 and (ii) a peptide containing the amino acid sequence of SEQ ID NO: 2.

According to another aspect of the present invention, there is provided a use, in the preparation of a medicine or food for inhibiting aging, of a composition which contains a peptide complex containing (i) a peptide containing the amino acid sequence of SEQ ID NO: 1 and (ii) a peptide containing the amino acid sequence of SEQ ID NO: 2.

In an embodiment, the inhibition of aging may be inhibition of cellular aging.

In an embodiment, the cell may be an adipocyte or myocyte.

In an embodiment, the cell may be an adipocyte or myocyte in a state of insulin metabolic disorder or in an inflammatory state.

In an embodiment, the insulin metabolic disorder may be a state of having insulin resistance. According to an aspect of the present invention, there is provided a method for activating sirtuin 1 (SIRT1), which includes administering to a subject in need of activation of SIRT1, a composition containing a peptide complex containing (i) a peptide containing the amino acid sequence of SEQ ID NO: 1 and (ii) a peptide containing the amino acid sequence of SEQ ID NO: 2 as an active ingredient.

According to another aspect of the present invention, there is provided a use, for the activation of sirtuin 1 (SIRT1), of a peptide complex, which contains (i) a peptide containing the amino acid sequence of SEQ ID NO: 1 and (ii) a peptide containing the amino acid sequence of SEQ ID NO: 2.

According to another aspect of the present invention, there is provided a composition for activating SIRT1, which contains as an active ingredient a peptide complex that contains (i) a peptide containing an amino acid sequence of SEQ ID NO: 1 and (ii) a peptide containing an amino acid sequence of SEQ ID NO: 2.

According to another aspect of the present invention, there is provided a use, in the preparation of a medicament or food for activating sirtuin 1 (SIRT1), of a peptide complex, which contains (i) a peptide containing an amino acid sequence of SEQ ID NO: 1 and (ii) a peptide containing an amino acid sequence of SEQ ID NO: 2.

### ADVANTAGEOUS EFFECTS

The peptide complex of the present invention promotes the expression of the SIRT1 gene, known as a longevity gene, promotes the expression of the FGF21 gene, and increases the phosphorylation of AMPK. In addition, the peptide complex of the present invention exhibits an anti-inflammatory effect. Therefore, the peptide complex of the present invention may be used for inhibiting aging, promoting the expression of the SIRT1 gene, which is a longevity gene, and treating or preventing SIRT1-related diseases.

However, the effects of the present invention are not limited to the effects mentioned above, and other effects not mentioned will be clearly understood by those skilled in the art from the description below.

### BRIEF DESCRIPTION OF THE DRAWINGS

FIGS. 1a and 1b show that in adipocytes, the phosphorylation of AMPKα is increased and the expression levels of SIRT1 and FGF21 proteins are increased by the treatment with the peptide complex of the present invention.
FIG. 2 shows that the expression level of FGF21 protein increased when adipocytes were treated with the peptide complex for 48 and 72 hours.
FIGS. 3a and 3b show that in adipocytes in a state of insulin metabolic disorder induced by palmitate treatment, the phosphorylation of AMPKα increased and the expression level of SIRT1 protein increased by the treatment with the peptide complex of the present invention.
FIGS. 4a and 4b show that the expression of fibroblast growth factor 21 (FGF21), sirtuin 1 (SIRT1), AMP-activated protein kinase (AMPK), and FOXO3A genes was increased in adipocytes in a state of insulin metabolic disorder induced by palmitate treatment by the treatment with the peptide complex of the present invention.
FIGS. 5a and 5b show that in adipocytes in which an inflammatory state was induced by palmitate treatment, the phosphorylation of IκBα was reduced and the expression level of TNFα was reduced by the treatment with the peptide complex of the present invention.
FIGS. 6a and 6b show that the phosphorylation of AMPK was increased and the expression levels of SIRT1 and FGF21 proteins were increased by the treatment with the peptide complex of the present invention in myocytes.
FIGS. 7a and 7b show that the phosphorylation of AMPK was increased and the expression level of SIRT1 protein was increased by the treatment with the peptide complex of the present invention in myocytes in a state of insulin metabolic disorder induced by palmitate treatment.

### MODE FOR CARRYING OUT THE INVENTION

Hereinafter, the present invention will be described in detail by way of examples. However, the following examples are intended to specifically illustrate the present invention, and the content of the present invention is not limited by the following examples.

### [Examples]

### Preparation Example 1: Preparation of peptides and peptide complexes

Using an automatic peptide synthesizer (Milligen 9050, Millipore, USA), peptides having amino acid sequences of SEQ ID NO: 1 and SEQ ID NO: 2 as shown in Table 1 below were synthesized, and these synthesized peptides were purified using C18 reverse-phase high-performance liquid chromatography (HPLC) (Waters Associates, USA). The column used was ACQUITY UPLC BEH300 C18 (2.1 mm X 100 mm, 1.7 µm, Waters Co, USA).

**[Table 1]**

| SEQ ID NO: | Amino Acid Sequence of Peptide |
|---|---|
| 1 | LKTRN |
| 2 | KGSATGWMA |

A peptide complex was prepared by mixing equal amounts of the peptide of SEQ ID NO: 1 and the peptide of SEQ ID NO: 2 prepared above, and its efficacy was evaluated.

### Experimental Example 1: Increased phosphorylation of AMPK and increased expression levels of SIRT1 and FGF21 in adipocytes

An experiment was performed to determine whether the peptide complex prepared in Preparation Example 1 using adipocytes increases the phosphorylation of AMP-activated protein kinase (AMPK), which is a longevity-related protein, and increases the expression levels of sirtuin 1 (SIRT1) and fibroblast growth factor 21 (FGF21) proteins in adipocytes.

When 3T3-L1 preadipocytes reached 80% confluence, they were seeded at 5 × 10³ cells/well in a 96-well plate and used for the experiment. Two days after reaching confluence, the medium was replaced with a differentiation-inducing medium to induce the differentiation into adipocytes, and two days after the induction, the cells were cultured for 2 days in a medium containing 10% FBS and 10 µg/mL insulin, and then the medium was replaced with 10% FBS every 2 days until their differentiation into adipocytes. Once the differentiation was complete, the medium was replaced with a serum-free medium and cultured for 4 hours to induce starvation. The peptide complexes were treated at concentrations of 0.2 µg/mL, 2 µg/mL, and 20 µg/mL, incubated for 1 hour, dissolved by adding dissolution buffer thereto, and the proteins obtained by centrifugation at 4°C and 12,000 rpm for 30 minutes were quantified using a BCA kit. The proteins were separated by sodium dodecyl sulfate-polyacrylamide gel electrophoresis (SDS-PAGE) and then electrotransferred onto a membrane. The membrane with the proteins attached was blocked by treating it with 5% skim milk, and then the primary antibody was reacted overnight at 4°C. After washing the resultant with PBS-T, the secondary antibody was reacted at room temperature for 1 hour, washed again with PBS-T, and visualized through a Western detection reagent (Elpis Biotech, Daejeon, Korea) using Gel Doc (Bio-Rad, Hercules, CA, USA). The antibodies used in the experiment are as follows: an anti-Phospho-AMPK antibody (Cell Signaling Technology, USA), an anti-SIRT1 antibody (Cell Signaling Technology, USA), an anti-FGF21 antibody (Abcam, UK), and an anti-β-actin antibody (Cell Signaling Technology, USA).

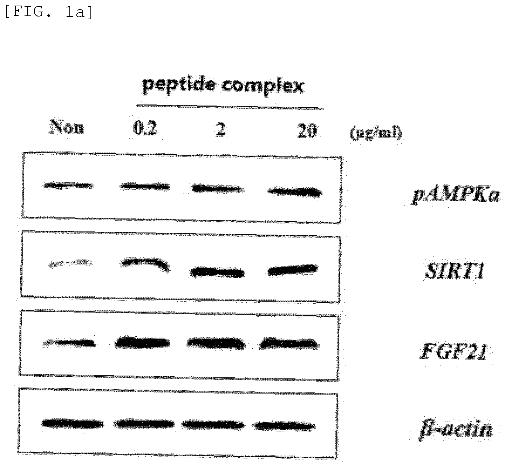

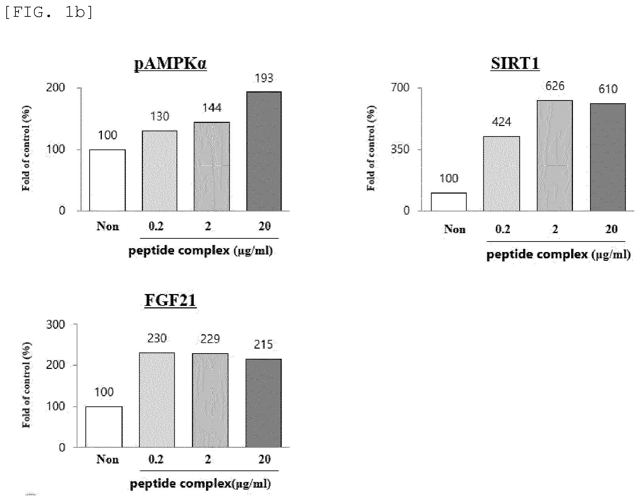

As a result of the experiment, as shown in FIGS. 1a and 1b, it was confirmed that in adipocytes, the phosphorylation of AMPKα was increased and the expression levels of SIRT1 and FGF21 proteins were increased by treating with the peptide complex of the present invention.

### Experimental Example 2: Increased expression level of FGF21 in adipocytes

An experiment was performed using adipocytes to determine whether the peptide complex prepared in Preparation Example 1 increases the expression level of FGF21 protein, a longevity-related protein, in adipocytes.

When 3T3-L1 preadipocytes reached 80% confluence, they were seeded at 5 × 10³ cells/well in a 96-well plate and used for the experiment. Two days after reaching confluence, the medium was replaced with a differentiation-inducing medium to induce the differentiation into adipocytes, and two days after the induction, the cells were cultured for 2 days in a medium containing 10% FBS and 10 µg/mL insulin, and then the medium was replaced with 10% FBS every 2 days until differentiation into adipocytes. Once the differentiation was complete, the medium was replaced with a serum-free medium and cultured for 4 hours to induce starvation. The peptide complexes were treated at concentrations of 0.2 µg/mL, 2 µg/mL, and 20 µg/mL, and cultured for 48 and 72 hours, respectively. The complexes were dissolved by adding dissolution buffer thereto, and the proteins obtained by centrifugation at 4°C and 12,000 rpm for 10 minutes were analyzed using a Quantikine ELISA Kit, and the absorbance was measured at 450 nm using a Microplate Reader.

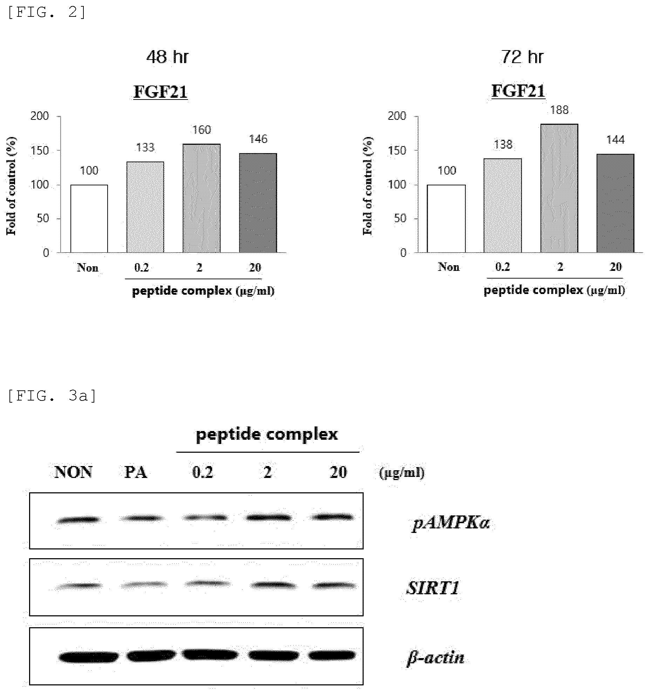

As shown in FIG. 2, the experimental results confirmed that the expression level of FGF21 protein increased when the peptide complex was treated in adipocytes for 48 and 72 hours.

### Experimental Example 3: Increased phosphorylation of AMPK and increased expression level of SIRT1 in adipocytes with insulin metabolic disorder induced by palmitate treatment

An experiment was performed to determine whether the peptide complex prepared in Preparation Example 1 increases the phosphorylation of AMP-activated protein kinase (AMPK) and the expression level of SIRT1 (sirtuin1) protein in adipocytes in a state of insulin metabolic disorder induced by palmitate treatment.

When 3T3-L1 preadipocytes reached 80% confluence, they were seeded at 5 × 10³ cells/well in a 96-well plate and used for the experiment. Two days after reaching confluence, the medium was replaced with a differentiation-inducing medium to induce the differentiation into adipocytes, and two days after the induction, the cells were cultured for 2 days in a medium containing 10% FBS and 10 µg/mL insulin, and then the medium was replaced with 10% FBS every 2 days until their differentiation into adipocytes. Once differentiation was complete, the medium was replaced with a serum-free medium and cultured for 4 hours to induce starvation. After 30 minutes of treatment with 25 µM palmitate, the peptide complexes were treated at concentrations of 0.2 µg/mL, 2 µg/mL, and 20 µg/mL and incubated for 24 hours. After dissolving by adding dissolution buffer, the proteins obtained by centrifugation at 4°C and 12,000 rpm for 30 minutes were quantified using a BCA kit. The proteins were separated by sodium dodecyl sulfate-polyacrylamide gel electrophoresis (SDS-PAGE) and then electrotransferred onto a membrane. The membrane with the proteins attached was blocked by treating it with 5% skim milk, and then the primary antibody was reacted overnight at 4°C. After washing the resultant with PBS-T, the secondary antibody was reacted for 1 hour at room temperature, washed again with PBS-T, and visualized through a Western detection reagent (Elpis Biotech, Daejeon, Korea) using Gel Doc (Bio-Rad, Hercules, CA, USA). The antibodies used in the experiment were as follows: an anti-Phospho-AMPK antibody (Cell Signaling Technology, USA), an anti-SIRT1 antibody (Cell Signaling Technology, USA), and an anti-β-actin antibody (Cell Signaling Technology, USA).

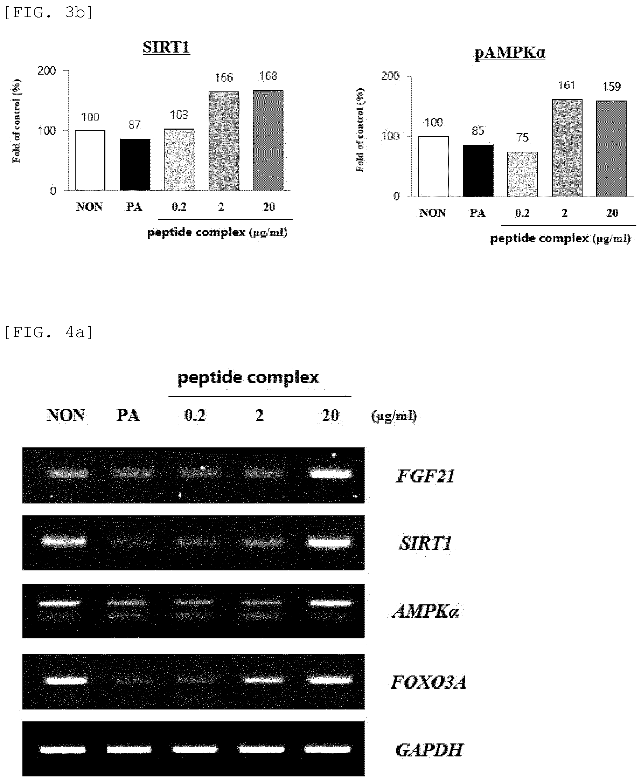

As a result of the experiment, as shown in FIGS. 3a and 3b, it was confirmed that in adipocytes in a state of insulin metabolic disorder induced by palmitate treatment, the phosphorylation of AMPKα increased and the expression level of SIRT1 protein increased by the treatment with the peptide complex of the present invention.

### Experimental Example 4: Increased expression levels of FGF21, SIRT1, and AMPK, FOXO3A genes in adipocytes with insulin metabolic disorder induced by palmitate treatment

An experiment was performed to determine whether the peptide complex prepared in Preparation Example 1 increases the gene expression of longevity-related genes, FGF21, SIRT1, AMPK, and FOXO3A, in adipocytes in a state of insulin metabolic disorder induced by palmitate treatment.

When 3T3-L1 preadipocytes reached 80% confluence, they were seeded at 5 × 10³ cells/well in a 96-well plate and used for the experiment. Two days after reaching confluence, the medium was replaced with differentiation-inducing medium to induce the differentiation into adipocytes, and two days after the induction, the medium was cultured for 2 days in a medium containing 10% FBS and 10 µg/mL insulin, and then the medium was replaced with 10% FBS every 2 days until their differentiation into adipocytes. After differentiation was complete, the medium was replaced with glucose-free medium, cultured for 16 hours, and then starvation was induced by culturing for 40 minutes in 100 µL KPPH buffer containing 2% BSA. After treating with 25 µM palmitate for 30 minutes, the peptide complexes were treated at concentrations of 0.2 µg/mL, 2 µg/mL, and 20 µg/mL, cultured for 24 hours, and RNA was extracted after 24 hours and RT-PCR was performed. The group treated with palmitate but not treated with the peptide complex was used as the control group (PA). RNA was extracted from 3T3-L1 cells using the easy-BLUETMTotal RNA extraction kit (Qiagen, Germany). The extracted RNA was converted to cDNA with RT-PCR premix (iNtRON Biotechnology, Seongnam, Korea). After preparing a reaction mixture containing a PCR premix (iNtRON Biotechnology, Seongnam, Korea) and primers for each gene of fibroblast growth factor 21 (FGF21), sirtuin 1 (SIRT1), AMP-activated protein kinase (AMPK), or GAPDH gene, PCR was performed using a PCR machine (Eppendorf, Germany). Subsequently, the mRNA expression pattern was determined by agarose gel electrophoresis. The base sequences of the primers used in the experiment are shown in Table 2 below.

**[Table 2]**

| Primer Name | Sequence (5'-> 3') | SEQ ID NO: |
|---|---|---|
| FGF21 Forward | 5'-CGT CTG CCT CAG AAG GAC TC-3' | 3 |
| FGF21 Reverse | 5' -TCT ACC ATG CTC AGG GGG TC-3' | 4 |
| SIRT1 Forward | 5' -GAT CCT TTG GAT TCC TGC AA-3' | 5 |
| SIRT1 Reverse | 5' -AGT TCC AGC CGT CTC TGT GT-3' | 6 |
| AMPK Forward | 5' -TCA CCG GAC ATA AAG TGG CT-3' | 7 |
| AMPK Reverse | 5' -TGA TGA TGT GAG GGT GCC TG-3' | 8 |
| FOXO3A Forward | 5' -CCC CAT CGG GGT TGA TGA TCC ACC-3' | 9 |
| FOXO3A Reverse | 5' - TTT GTC CCA GAT CTA CGA GTG-3' | 10 |
| GAPDH Forward | 5' - GTG ATG GCA TGG ACT GTG GT-3' | 11 |
| GAPDH Reverse | 5' - GGA GCC AAA AGG GTC ATC AT-3' | 12 |

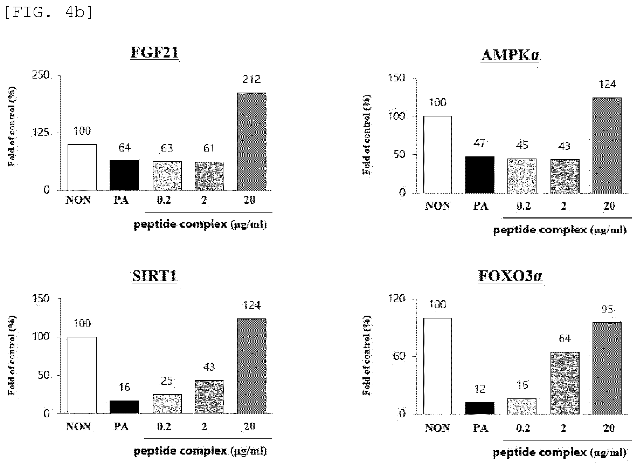

As shown in FIGS. 4a and 4b, the experimental results showed that the expression of fibroblast growth factor 21 (FGF21), sirtuin 1 (SIRT1), AMP-activated protein kinase (AMPK), and FOXO3A genes increased in adipocytes in a state of insulin metabolic disorder induced by palmitate treatment by the peptide complex treatment of the present invention.

### Experimental Example 5: Anti-inflammatory effect in inflammatory response state induced by palmitate treatment

An experiment was performed to determine whether the peptide complex prepared in Preparation Example 1 induces a decrease in the phosphorylation of IκBα and a decrease in the expression level of the inflammatory cytokine TNFα in adipocytes under an inflammatory state induced by palmitate treatment.

When 3T3-L1 preadipocytes reached 80% confluence, they were seeded at 5 × 10³ cells/well in a 96-well plate and used for the experiment. Two days after reaching confluence, the medium was replaced with a differentiation-inducing medium to induce the differentiation into adipocytes. Two days after the induction, the cells were cultured for 2 days in a medium containing 10% FBS and 10 µg/mL insulin, and then the medium was replaced with 10% FBS every 2 days until their differentiation into adipocytes. Once the differentiation was complete, the medium was replaced with a serum-free medium and cultured for 4 hours to induce starvation. After treating with 25 µM palmitate for 30 minutes, the peptide complexes were treated at concentrations of 0.2 µg/mL, 2 µg/mL, and 20 µg/mL and incubated for 24 hours. The control group (PA) was treated only with palmitate but not with the peptide complex. After dissolving by adding dissolution buffer thereto, the proteins obtained by centrifugation at 4°C and 12,000 rpm for 30 minutes were quantified using a BCA kit. The proteins were subjected to sodium dodecyl sulfate-polyacrylamide gel electrophoresis (SDS-PAGE) and then electrotransferred onto a membrane. The membrane with the proteins attached was blocked by treating it with 5% skim milk, and then the primary antibody was reacted overnight at 4°C. After washing with PBS-T, the secondary antibody was reacted for 1 hour at room temperature, washed again with PBS-T, and visualized through a Western detection reagent (Elpis Biotech, Daejeon, Korea) using Gel Doc (Bio-Rad, Hercules, CA, USA). The antibodies used in the experiment are as follows: an anti-Phospho-IκBα antibody (Cell Signaling Technology, USA), an anti-TNFα antibody (Cell Signaling Technology, USA), an anti-β-actin antibody (Cell Signaling Technology, USA).

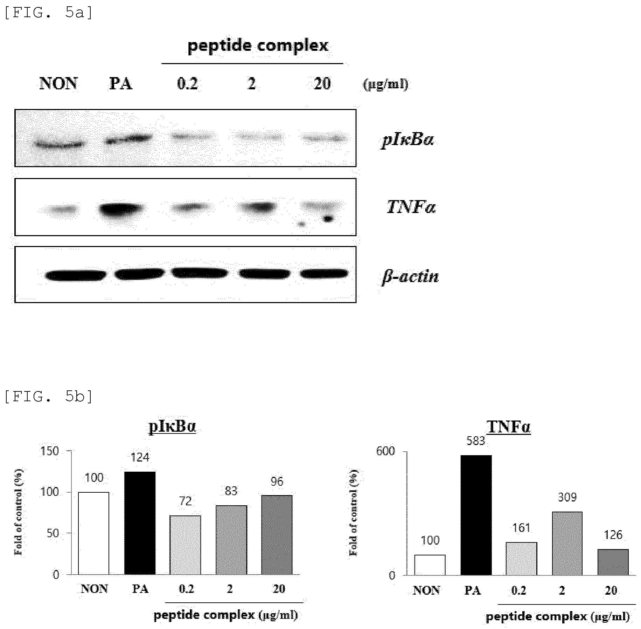

As shown in FIGS. 5a and 5b, the experimental results showed that in adipocytes in which inflammation was induced by palmitate treatment, the phosphorylation of IκBα was reduced and the expression level of TNFα was reduced by the treatment with the peptide complex of the present invention.

### Experimental Example 6: Increased AMPK phosphorylation and increased SIRT1 and FGF21 protein expression in myocytes

An experiment was performed to determine whether the peptide complex increases the phosphorylation of AMPK and the expression of SIRT1 and FGF21 proteins in myocytes.

C2C12 cells, mouse-derived myoblasts, were cultured in DMEM medium containing 1% P/S and 2% BCS. When the cell confluency reached 70-80%, the cells were seeded in a 6-well culture plate. When the cell confluency in the 6-well culture plate reached 100%, the medium was replaced with differentiation medium (1% P/S, 2% horse serum), and thereafter, the differentiation medium (1% P/S, 2% horse serum) was replaced once every two days. Five days after the first replacement, the peptide complex was treated at concentrations of 0.2 µg/mL, 2 µg/mL, and 20 µg/mL and cultured for 24 hours. The group untreated with the peptide complex was used as the control. After dissolving by adding dissolution buffer thereto, the proteins obtained were centrifuged at 4°C and 12,000 rpm for 30 minutes, and quantified using a BCA kit. The proteins were subjected to sodium dodecyl sulfate-polyacrylamide gel electrophoresis (SDS-PAGE) and then electrotransferred onto a membrane. The membrane with the proteins attached was blocked by treating it with 5% skim milk, and then the primary antibody was reacted overnight at 4°C. After washing with PBS-T, the secondary antibody was reacted for 1 hour at room temperature, washed again with PBS-T, and visualized through a Western detection reagent (Elpis Biotech, Daejeon, Korea) using Gel Doc (Bio-Rad, Hercules, CA, USA). The antibodies used in the experiments are as follows: an anti-Phospho-AMPK antibody (Cell signaling technology, USA), an anti-SIRT1 antibody (Cell signaling technology, USA), anti-FGF21 antibody (Abcam, UK), an anti-β-actin antibody (Cell signaling technology, USA).

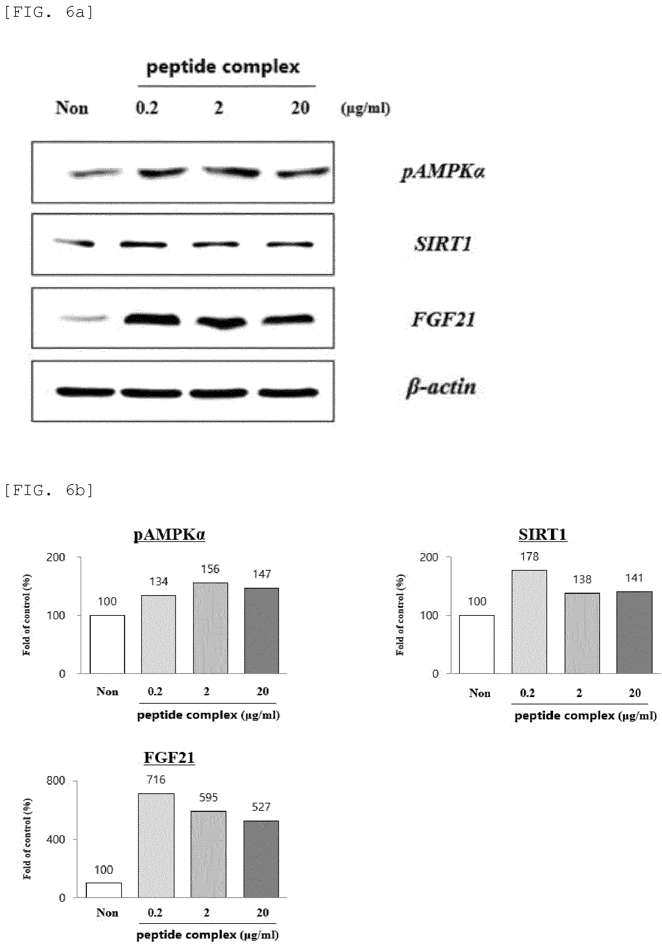

As shown in the experimental results in FIGS. 6a and 6b, it was confirmed that the phosphorylation of AMPK increased and the expression levels of SIRT1 and FGF21 proteins increased by the treatment with the peptide complex of the present invention in myocytes.

### Experimental Example 7: Increased AMPK phosphorylation and increased SIRT1 expression level in myocytes with insulin metabolic disorder induced by palmitate treatment

An experiment was performed to determine whether the peptide complex increases AMPK phosphorylation and SIRT1 expression levels in myocytes in a state of insulin metabolic disorder induced by palmitate treatment.

C2C12 cells, mouse-derived myoblasts, were cultured in DMEM medium containing 1% P/S and 2% BCS. When the cell confluency reached 70-80%, the cells were seeded in a 6-well culture plate. When the cell confluency in the 6-well culture plate reached 100%, the medium was replaced with differentiation medium (1% P/S, 2% horse serum), and thereafter, the differentiation medium (1% P/S, 2% horse serum) was replaced once every two days. Five days after the first replacement, the peptide complex was treated at concentrations of 0.2 µg/mL, 2 µg/mL, and 20 µg/mL and cultured for 24 hours. The group untreated with the peptide complex was used as the control. After dissolving by adding dissolution buffer thereto, the proteins obtained were centrifuged at 4°C and 12,000 rpm for 30 minutes, and quantified using a BCA kit. The proteins were subjected to sodium dodecyl sulfate-polyacrylamide gel electrophoresis (SDS-PAGE) and then electrotransferred onto a membrane. The membrane with the proteins attached was blocked by treating it with 5% skim milk, and then the primary antibody was reacted overnight at 4°C. After washing with PBS-T, the secondary antibody was reacted for 1 hour at room temperature, washed again with PBS-T, and visualized through a Western detection reagent (Elpis Biotech, Daejeon, Korea) using Gel Doc (Bio-Rad, Hercules, CA, USA). The antibodies used in the experiments are as follows: an anti-Phospho-AMPK antibody (Cell signaling technology, USA), an anti-SIRT1 antibody (Cell signaling technology, USA), an anti-β-actin antibody (Cell signaling technology, USA).

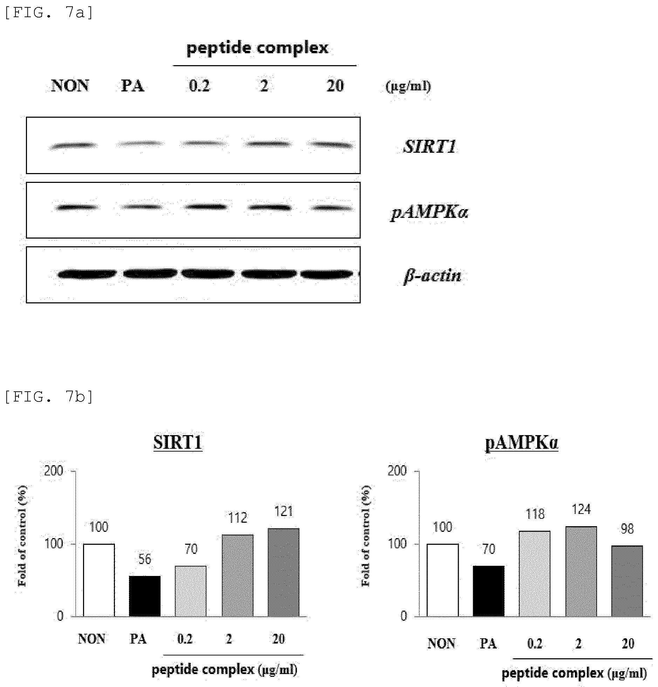

As a result of the experiment, as shown in FIGS. 7a and 7b, it was confirmed that the phosphorylation of AMPK was increased and the expression level of SIRT1 protein was increased by the treatment with the peptide complex of the present invention in myocytes in a state of insulin metabolic disorder induced by palmitate treatment.

### Preparation Example 2: Preparation of pharmaceutical composition

### 2-1. Preparation of powders

2 g of the peptide complex of the present invention
1 g of lactose

The ingredients were mixed and filled into a sealed bag to prepare a powder preparation.

### 2-2. Preparation of tablets

100 mg of the peptide complex of the present invention
100 mg of corn starch
100 mg of lactose
2 mg of magnesium stearate

The ingredients were mixed and tableted according to a conventional method for manufacturing tablets to prepare tablets.

### 2-3. Preparation of capsules

100 mg of the peptide complex of the present invention
100 mg of corn starch
100 mg of lactose
2 mg of magnesium stearate

The ingredients were mixed and the mixture was filled into a gelatin capsule according to a conventional method for manufacturing capsules to prepare capsules.

### 2-4. Preparation of pills

1 g of the peptide complex of the present invention
1.5 g of lactose
1 g of glycerin
0.5 g of xylitol

The ingredients were mixed and the mixture was prepared into pills (4 g/pill) according to a conventional method.

### 2-5. Preparation of granules

150 mg of the peptide complex of the present invention
50 mg of a soybean extract
200 mg of glucose
600 mg of starch

The ingredients were mixed and 100 mg of 30% ethanol was added thereto, and the mixture was dried at 60°C to form granules, and then filled into pouches.

### Preparation Example 3: Preparation of functional food composition

### 3-1. Preparation of health food products

500 µg of the peptide complex of the present invention
an adequate amount of a vitamin mixture
70 mg of vitamin A acetate
1.0 mg of vitamin E
0.13 mg of vitamin D
0.15 mg of vitamin B2
0.5 mg of vitamin B6
0.2 mg of vitamin B12
10 mg of vitamin C
10 mg of biotin
1.7 mg of nicotinamide
50 mg of folic acid
0.5 mg of calcium pantothenate
an adequate amount of a mineral mixture
1.75 mg of ferrous sulfate
0.82 mg of zinc oxide
25.3 mg of magnesium carbonate
15 mg of potassium phosphate monobasic
55mg of calcium phosphate dibasic
90 mg of potassium citrate
100 mg of calcium carbonate
24.8 mg of magnesium chloride

The composition ratio of the vitamins and a mineral mixture is a preferred embodiment of a mixture of ingredients suitable for relatively healthy foods, but the mixing ratio may be arbitrarily modified, and the ingredients may be mixed according to a conventional health food manufacturing method, and then granules may be prepared therefrom and used to prepare a health food composition according to a conventional method.

### 3-2. Preparation of health drinks

500 µg of the peptide complex of the present invention
1000 mg of citric acid
100 g of an oligosaccharide
2 g of a plum concentrate
1 g of taurine
add purified water to a final volume of 900 mL

According to the conventional health drink manufacturing method, the ingredients were mixed, stirred and heated at 85°C for about 1 hour, and the resulting solution was filtered, placed in a sterilized container, sealed and sterilized, and then stored in the refrigerator, and then used to manufacture a health drink composition. The composition ratio is a preferred embodiment of a mixture of ingredients relatively suitable for a favorite drink, but the mixing ratio can be arbitrarily modified according to regional or ethnic preferences such as target class, demanding country, and intended use.

Although the representative embodiments of the present application have been described above as embodiments, the scope of the present application is not limited to the specific embodiments described above, and those with ordinary knowledge in the relevant field will be able to make appropriate changes within the scope described in the claims of the present application.

## Claims

1. An anti-aging composition comprising, as an active ingredient, a peptide complex comprising (i) a peptide comprising an amino acid sequence of SEQ ID NO: 1 and (ii) a peptide comprising an amino acid sequence of SEQ ID NO: 2.

2. The anti-aging composition of claim 1, wherein the peptide complex increases an expression level of sirtuin 1 (SIRT1) protein, or fibroblast growth factor 21 (FGF21) protein or increases phosphorylation of AMP-activated protein kinase (AMPK) in a cell.

3. The anti-aging composition of claim 1, wherein the peptide complex increases an expression of sirtuin 1 (SIRT1) gene, fibroblast growth factor 21 (FGF21) gene, or AMP-activated protein kinase (AMPK) gene in a cell.

4. The anti-aging composition of claim 1, wherein the peptide complex induces a decrease in IkBα phosphorylation or a decrease in the expression level of the inflammatory cytokine TNFα in cells in an inflammatory response state.

5. The anti-aging composition of claim 1, wherein the anti-aging composition is a pharmaceutical composition for the purpose of inhibiting aging.

6. The anti-aging composition of claim 1, wherein the anti-aging composition is a food composition for use in the inhibition of aging.

7. The anti-aging composition of claim 1, wherein the anti-aging composition is a cosmetic composition for use in the inhibition of aging.

8. The anti-aging composition of any one of claims 1 to 7, wherein the aging is cell aging induced by oxidative stress.

9. The anti-aging composition of any one of claims 1 to 7, wherein the aging is aging in adipocytes or myocytes.

10. A composition for activating sirtuin 1 (SIRT1), comprising as an active ingredient a peptide complex comprising (i) a peptide comprising an amino acid sequence of SEQ ID NO: 1 and (ii) a peptide comprising an amino acid sequence of SEQ ID NO: 2.

11. A pharmaceutical composition for treating or preventing a sirtuin 1 (SIRT1)-related disease, comprising as an active ingredient a peptide complex comprising (i) a peptide comprising an amino acid sequence of SEQ ID NO: 1 and (ii) a peptide comprising an amino acid sequence of SEQ ID NO: 2.

12. The pharmaceutical composition of claim 11, wherein the sirtuin 1 (SIRT1)-related disease is a disease selected from the group consisting of metabolic diseases, cancers, and neurodegenerative diseases.

13. The pharmaceutical composition of claim 12, wherein:
the metabolic disease is a disease selected from the group consisting of insulin-resistant diabetes, adult-onset diabetes, diabetic nephropathy, type 2 diabetes, obesity, impaired glucose tolerance, hypercholesterolemia, dyslipidemia, hyperlipidemia, microvascular dysfunction, and arteriosclerosis;
the cancer is a cancer selected from the group consisting of blood cancer, breast cancer, head or neck cancer, uterine cancer, cervical cancer, ovarian cancer, lung cancer, bladder cancer, esophageal cancer, mesothelioma, neuroblastoma, testicular cancer, leukemia, stomach cancer, liver cancer, colon cancer, skin cancer, brain cancer, laryngeal cancer, prostate cancer, thyroid cancer, kidney cancer, and rectal cancer; and
the neurodegenerative disease is a disease selected from the group consisting of Alzheimer's disease, Huntington's disease, Parkinson's disease, amyotrophic lateral sclerosis, multiple sclerosis, diseases caused by polyglutamine aggregation, AIDS-related dementia, sensory neuropathy, autonomic neuropathy, motor neuropathy, and retinopathy.
